# EUROPEAN PATENT SPECIFICATION

(11) **EP 1 962 724 B1**
(45) Date of publication and mention of the grant of the patent: **02.01.2013**
(21) Application number: 06848518.4
(22) Date of filing: 30.11.2006
(51) Int. Cl.: A61F 2/16

(54) **HYDRAULIC ACCOMMODATING INTRAOCULAR LENS**
HYDRAULISCHE UND AKKOMODATIONSFÄHIGE INTRAOKULARLINSE
LENTILLE INTRAOCULAIRE D'ACCOMMODATION HYDRAULIQUE

(30) Priority: 07.12.2005 US 297232
(43) Date of publication of application: 03.09.2008
(73) Proprietor: C&C Vision International Limited, Dublin 2 (IE)
(72) Inventor: CUMMING, J. Stuart, Aliso Viejo, California 92656 (US)
(74) Representative: Walcher, Armin
(86) International application number: PCT/US2006/061428
(87) International publication number: WO 2007/067867

(56) References cited:
- WO-A-01/97742
- WO-A-96/25126
- WO-A-2004/046768

## Description

Intraocular lenses have for many years had a design of a single optic with loops attached to the optic to center the lens and fixate it in the empty capsular bag of the human lens. In the mid '80s plate lenses were introduced, which comprised a silicone lens, 10.5 mm in length, with a 6 mm optic. These lenses could be folded but did not fixate well in the capsular bag, but resided in pockets between the anterior and posterior capsules. The first foldable lenses were all made of silicone. In the mid 1990s an acrylic material was introduced as the optic of lenses. The acrylic lens comprised a biconvex optic with a straight edge into which were inserted loops to center the lens in the eye and fixate it within the capsular bag.

Recently accommodating intraocular lenses have been introduced to the market, which generally are modified plate haptic lenses and, like the silicone plate haptic lenses, have no clear demarcation between the junction of the plate with the optic's posterior surface. A plate haptic lens may be referred to as an intraocular lens having two or more plate haptics joined to the optic.

Flexible acrylic material has gained significant popularity among ophthalmic surgeons. In 2003 more than 50% of the intraocular lenses implanted had acrylic optics. Hydrogel lenses have also been introduced. Both the acrylic and hydrogel materials are incapable of multiple flexions without fracturing.

The advent of an accommodating lens which functions by moving along the axis of the eye by repeated flexions somewhat limited the materials from which the lens could be made. Silicone is the ideal material, since it is flexible and can be bent probably several million times without showing any damage. Additionally a groove or hinge can be placed across the plate adjacent to the optic as part of the lens design to facilitate movement of the optic relative to the outer ends of the haptics. On the other hand, acrylic material fractures if it is repeatedly flexed.

An example accommodating lens is a type as disclosed in U.S. Patent No. 6,387,126 and others in the name of J. Stuart Cumming. An accommodating intraocular lens according to the preamble of claim 1 is known from the document WO-A-2004/046768.

### SUMMARY OF THE INVENTION

According to a preferred embodiment of this invention, an accommodating lens comprises a lens with a flexible solid and interior liquid optic, preferably with two or more extended portions from the solid optic which may be plate haptics capable of multiple flexions without breaking, preferably along with fixation and centration features at their distal ends. There may be a hinge or groove across the extended portions adjacent to the optic to facilitate the anterior and posterior movement of the optic relative to the outer ends of the extended portions. On the other hand, the optic may be rigidly attached to the haptics. Also, haptics can be omitted.

According to the present invention the optic is of a foldable, flexible silicone, with an interior of liquid silicone, and the haptics are of a foldable material that will withstand multiple foldings without damage, e.g., silicone. Preferably, the end of the plate haptics have T-shaped fixation devices and the haptics are hinged to the optic.

The lens of the present invention is made of solid silicone with liquid silicone both of which have the same refractive index, and have a specific gravity the same as or very similar to that of the aqueous solution of the natural eye. The power of the lens, before implantation into the eye, can be changed by (1) changing the radius of a posterior portion of the optic, and/or (2) by changing the volume of the liquid silicone in the lens optic during the manufacturing process. During accommodation with contraction of the ciliary muscle and an increase in the vitreous cavity pressure the posterior surface of the solid silicone portion of the lens is pushed forward. This causes bulging of a thin anterior membrane thereby increasing its curvature, thus decreasing the radius of the anterior surface of the lens, for near vision. Also, the increase in vitreous cavity pressure can tilt the lens to further facilitate accommodation.

Accordingly, features of the present invention are to provide an improved form of accommodating lens formed from solid and liquid silicone.

### BRIEF DESCRIPTION OF THE DRAWINGS

Fig. 1 is a side cross sectional view of the preferred embodiment of the lens of the present invention.
Fig. 2 is a plan view from the posterior side of the lens.
Fig. 3 is a plan view from the anterior side of the lens.
Fig. 4 is a cross-sectional view of the lens like Fig. I but showing bulging or increased curvature of an anterior portion of the lens.

### DESCRIPTION OF THE PREFERRED EMBODIMENT

Turning now to the drawings, a preferred embodiment is shown in detail, comprising an intraocular lens with an optic 10 and haptics 16. The optic 10 is formed of two components, namely, a flexible solid portion 12 (12a - 12d) made of silicone, and an interior liquid silicone portion 14. The portions 12a and 12b are sufficiently solid to prevent deformation of the optic 10 upon implantation into the fibrosed capsular bag of the eye. The flexible extending portions 16 may be plate haptics which are capable of multiple flexations without damage, and formed, for example, of silicone. The optic 10 and haptics 16 preferably are uniplanar, and two or more haptics 16 extend distally from opposite sides of the optic 10. The outer ends of the haptics 16 may include flexible fingers 17 such as disclosed in US Patent No. 6,387,126 to Cumming. Preferably the edge 24 of the optic is a 360° square edge.

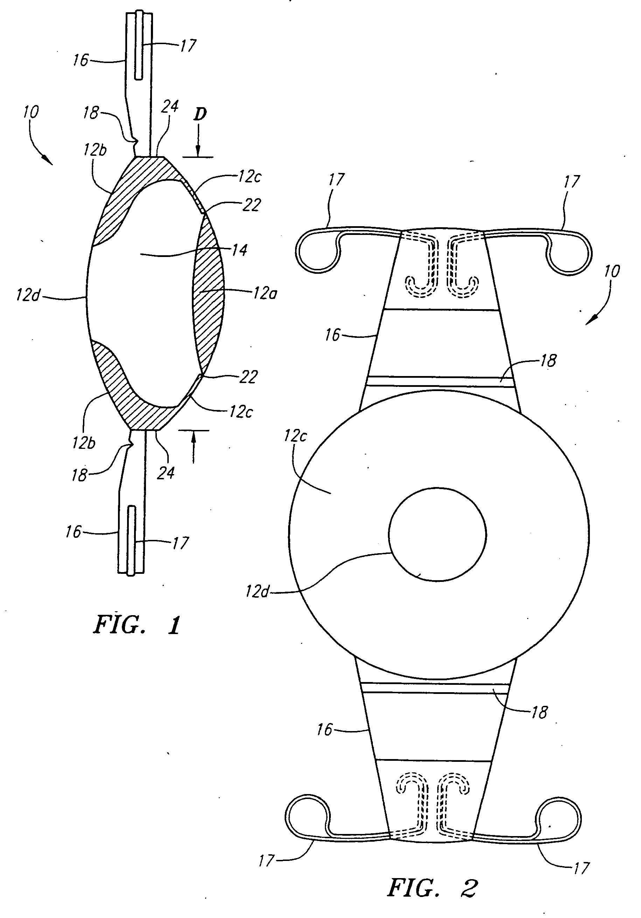

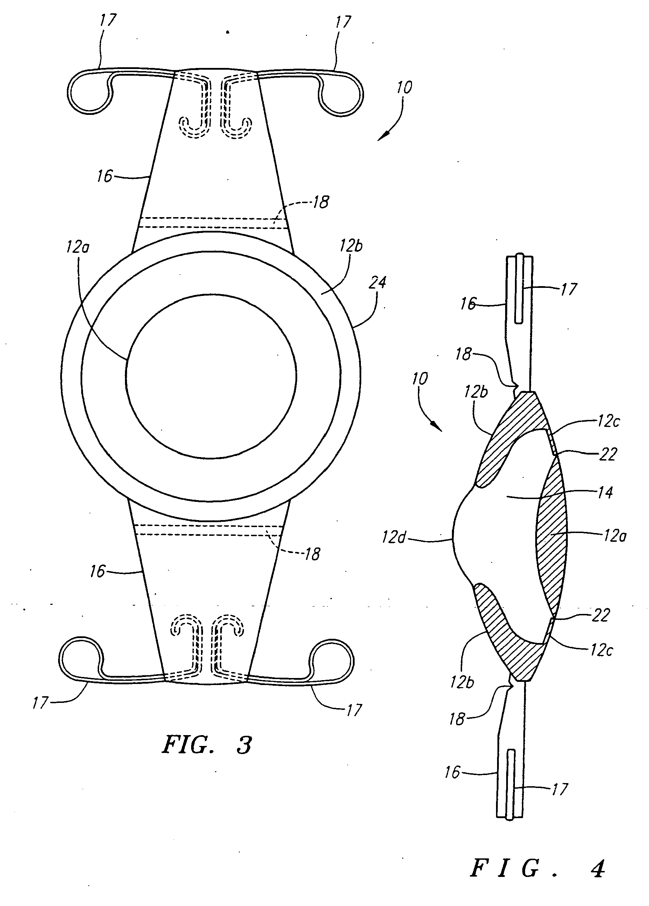

The lens 10 includes portions 12a, 12b and 12d of solid silicone and wherein the portion 12c is substantially thinner, and 12 d is even thinner than 12c, to enable a degree of flexibility as can be seen in comparing Fig. 1 and Fig. 4. The interior 14 is of liquid silicone. As is known, the specific gravity of the silicone used in this lens can be the same as or very similar to that of the acqueous solution in the human eye. This results in either no or negligible deformation of the liquid portion of the lens by gravity. The liquid silicone 14 has the same or similar refractive index as the solid components 12. The solid posterior radius of portion 12a prevents deformation of the posterior refracting surface. The radius of the portion 12a or 12b can be changed, during manufacturing, to select the desired power for the lens. Also, the power can be changed, during manufacturing, by changing the volume of the liquid silicone 14 in the lens optic 10.

In accommodating, the posterior surface portion 12a is pushed forward (to the left in Figs. 1 and 4) by vitreous cavity pressure with constriction of the ciliary muscle. The anterior portion 12d bulges with increased curvature, that is decreased radius, of the anterior portion 12d such as illustrated in Fig. 4.

Example dimensions are 4.5 - 10.5 mm in overall diameter of portion 12b from D to D in Fig. 1, up to a 5 mm diameter portion 12d, and a 3-6 mm thickness (from right to left) in Fig. 1. A typical thickness for the solid silicone portions 12a and 12b is between 0.5 mm and 1.5 mm. The thickness of the anterior membrane 12d is very thin, preferably about that of a toy balloon, and the thickness of the annulus 12c is approximately two times that thickness to give sufficient flexibility to the lens. The thickness at the hinge 18 area can be 0.1 mm. The hinge area 18 can be a "V" shape as shown but can be a square groove. Also, hinges 22 preferably are provided between 12c and 12a to facilitate anterior movement of the posterior optic 12a.

Furthermore, the power of the present lens can be changed after implantation in the eye by either injecting or removing liquid silicone from the optic 10.

The diameter of the portion 12d as well as its area can be less or more than that of the posterior portion 12a, dependent on the refractive range desired in the design of the lens.

As is well known in the art, an intraocular lens is implanted in the capsular bag of the eye after removal of the natural lens. The lens is inserted into the capsular bag by a generally circular opening cut in the anterior capsular bag of the human lens and through a small opening in the cornea or sclera. The outer ends of the haptics, or loops, are positioned in the cul-de-sac of the capsular bag. The outer ends of the haptics, or the loops, are in close proximity with the bag cul-de-sac, and in the case of any form of loops, the loops are deflected. Knobs can be provided on the outer end portions of the loops for improved securement in the capsular bag or cul-de-sac by engagement with fibrosis, which develops in the capsular bag following the surgical removal of the central portion of the anterior capsular bag.

As noted above, the haptics 16 may have a space or thin area 18 forming a hinge across their surface adjacent to the optic. This facilitates movement of the optic anteriorly and posteriorly relative to the outer ends of the haptics.

Accordingly, there has been shown and described a lens that comprises an optic of solid and liquid silicone and haptic plates, preferably with fixation fingers at the ends of each haptic.

Various changes, modifications, variations, and other uses and applications of the subject invention will become apparent to those skilled in the art after considering this specification together with the accompanying drawings and claims.

## Claims

1. An accommodating intraocular lens having an optic (10) formed of solid silicone and liquid silicone having the same or similar refractive index and have a specific gravity the same as or very similar to that of the aqueous solution of the natural eye,
**characterised by** the optic being circular and having a posterior portion of solid silicone (12a-d) extending to an anterior annular portion of solid silicone forming an outer diameter portion extending from the posterior side to an anterior side of the lens, the optic further comprising a solid central posterior portion (12c) extending between the posterior annular portion and the outer diameter of the solid central posterior portion by a membrane portion which is substantially thinner than the solid posterior and solid annular portions, and capable of deformation, the optic having a thin central anterior membrane portion (12d), and a liquid silicone (14) within the optic retained therein by the aforesaid portions, the optic being designed so that the thin central anterior membrane portion can change in radius of curvature upon contraction of the ciliary muscle causing an increase in vitreous cavity pressure on the posterior solid portion.

2. The lens as in Claim 1 further including extending portions from an edge of the optic for facilitating placement of the lens in an eye.

3. The lens as in Claim 1 whereby the optic can move anteriorly and posteriorly relative to the outer ends of the extending portions.

4. The lens as in Claim 1 wherein the solid silicone and the liquid silicone have approximately the same specific gravity as the aqueous solution of a human eye.

5. The lens as in Claim 1 designed such that compression of the posterior surface of the posterior portion by vitreous pressure can cause a symmetrical bulging of the central anterior membrane surface of the anterior portion.

6. The lens as in Claim 1 wherein the solid and liquid portions of the optic have substantially the same index of refraction.

7. The lens as in Claim 1 wherein the power of the optic can be changed after implantation by changing the amount of liquid silicone in the optic.

8. A lens as in Claim 1 wherein the central anterior membrane portion has a smaller area than the posterior portion.

9. The lens as in Claim 1 wherein the central anterior thin membrane portion has a larger area than the posterior portion.

10. A lens as in Claim 1 wherein the anterior membrane is thinner than the posterior annular membrane.

11. A lens as in Claim 1 wherein the anterior membrane is larger than the posterior annular membrane.

12. A lens as in Claim 3 wherein the extending portions are haptics and have a hinge adjacent to the optic.

13. A lens as in Claim 1 wherein the posterior solid central portion has a fixed central radius and the central anterior radius is deformable.

14. A lens as in Claim 1 wherein a solid peripheral area continues to join at a hinged junction with the solid posterior portion.

## Patentansprüche

1. Akkommodierende Intraokularlinse mit einer Optik (10), die aus festem Silicon und flüssigem Silicon gebildet ist, die den gleichen oder einen ähnlichen Brechungsindex aufweisen und das gleiche oder ein sehr ähnliches spezifisches Gewicht wie die wässrige Lösung des natürlichen Auges aufweisen,
**dadurch gekennzeichnet, dass** die Optik kreisförmig ist und einen hinteren Bereich aus festem Silicon (12a-d) aufweist, der sich bis zu einem vorderen ringförmigen Bereich aus festem Silicon erstreckt, der einen äußeren Durchmesserbereich bildet, der sich von der hinteren Seite zu einer vorderen Seite der Linse erstreckt, wobei die Optik ferner einen festen, zentralen, hinteren Bereich (12c) aufweist, der sich zwischen dem hinteren ringförmigen Bereich und dem äußeren Durchmesser des festen, zentralen, hinteren Bereiches erstreckt mittels eines Membranbereichs, der wesentlich dünner als der feste hintere Bereich und der feste ringförmige Bereich ist und deformierbar ist, wobei die Optik einen dünnen, zentralen, vorderen Membranbereich (12d) und ein flüssiges Silicon (14) innerhalb der Optik, das durch die vorgenannten Bereiche darin gehalten wird, aufweist, wobei die Optik so konstruiert ist, dass der dünne, zentrale, vordere Membranbereich seinen Krümmungsradius bei Kontraktion des Ziliarmuskels ändern kann, was eine Erhöhung des Drucks des Glaskörperraums auf den hinteren, festen Bereich bewirkt.

2. Linse nach Anspruch 1, ferner umfassend Erweiterungsbereiche, die vom Rand der Optik ausgehen, um die Platzierung der Linse in einem Auge zu erleichtern.

3. Linse nach Anspruch 1, wobei die Optik sich relativ zu den äußeren Enden der Erweiterungsbereiche nach vorne und nach hinten bewegen kann.

4. Linse nach Anspruch 1, wobei das feste Silicon und das flüssige Silicon annähernd das gleiche spezifische Gewicht wie die wässrige Lösung eines menschlichen Auges aufweisen.

5. Linse nach Anspruch 1, die so konstruiert ist, dass eine Kompression der hinteren Oberfläche des hinteren Bereiches durch den Druck des Glaskörpers ein symmetrisches Wölben der zentralen, vorderen Membranoberfläche des vorderen Bereiches verursachen kann.

6. Linse nach Anspruch 1, wobei die festen und flüssigen Bereiche der Optik im wesentlichen den gleichen Brechungsindex aufweisen.

7. Linse nach Anspruch 1, wobei die Stärke der Optik nach der Implantation durch Veränderung der Menge des flüssigen Silicons in der Optik verändert werden kann.

8. Linse nach Anspruch 1, wobei der zentrale, vordere Membranbereich eine kleinere Fläche als der hintere Bereich aufweist.

9. Linse nach Anspruch 1, wobei der zentrale, vordere, dünne Membranbereich eine größere Fläche als der hintere Bereich aufweist.

10. Linse nach Anspruch 1, wobei die vordere Membran dünner als die hintere, ringförmige Membran ist.

11. Linse nach Anspruch 1, wobei die vordere Membran größer als die hintere, ringförmige Membran ist.

12. Linse nach Anspruch 3, wobei die Erweiterungsbereiche Haptiken sind und ein neben der Optik angeordnetes Gelenk aufweisen.

13. Linse nach Anspruch 1, wobei der hintere, feste, zentrale, Bereich einen festen, zentralen Radius aufweist und der zentrale, vordere Radius deformierbar ist.

14. Linse nach Anspruch 1, wobei ein fester, peripherer Bereich sich fortsetzt, um an einer Gelenkverbindung eine Verbindung mit dem festen, hinteren Bereich herzustellen.

## Revendications

1. Lentille intraoculaire d'accommodation ayant une optique (10) constituée de silicone solide et de silicone liquide ayant un indice de réfraction identique ou similaire et ayant une densité identique ou très similaire à celle de la solution aqueuse de l'oeil naturel,
**caractérisée en ce que** l'optique est circulaire et comporte une partie postérieure de silicone solide (12a à d) s'étendant vers une partie annulaire antérieure de silicone solide formant une partie de diamètre extérieur s'étendant du côté postérieur vers un côté antérieur de la lentille, l'optique comprenant en outre une partie postérieure centrale solide (12c) s'étendant entre la partie annulaire postérieure et le diamètre extérieur de la partie postérieure centrale solide par une partie de membrane qui est sensiblement plus mince que les parties postérieure solide et annulaire solide, et capable de se déformer, l'optique comportant une mince partie de membrane antérieure centrale (12d), et une silicone liquide (14) dans l'optique retenu dans celle-ci par les parties susmentionnées, l'optique étant conçue de sorte que la mince partie de membrane antérieure centrale puisse changer de rayon de courbure lors de la contraction du muscle ciliaire provoquant une augmentation de la pression de la cavité vitreuse sur la partie solide postérieure.

2. Lentille selon la revendication 1, comprenant en outre des parties s'étendant d'un bord de l'optique pour faciliter le placement de la lentille dans un oeil.

3. Lentille selon la revendication 1, dans laquelle l'optique peut se déplacer antérieurement et postérieurement par rapport aux extrémités extérieures des parties étendues.

4. Lentille selon la revendication 1, dans laquelle la silicone solide et la silicone liquide ont approximativement la même densité que la solution aqueuse d'un oeil humain.

5. Lentille selon la revendication 1, conçue de sorte qu'une compression de la surface postérieure de la partie postérieure par la pression vitreuse puisse provoquer un bombement symétrique de la surface de membrane antérieure centrale de la partie antérieure.

6. Lentille selon la revendication 1, dans laquelle les parties solide et liquide de l'optique ont sensiblement le même indice de réfraction.

7. Lentille selon la revendication 1, dans laquelle la puissance de l'optique peut être modifiée après l'implantation en modifiant la quantité de silicone liquide dans l'optique.

8. Lentille selon la revendication 1, dans laquelle la partie de membrane antérieure centrale a une aire plus petite que celle de la partie postérieure.

9. Lentille selon la revendication 1, dans laquelle la mince partie de membrane antérieure centrale a une aire plus grande que celle de la partie postérieure.

10. Lentille selon la revendication 1, dans laquelle la membrane antérieure est plus mince que la membrane annulaire postérieure.

11. Lentille selon la revendication 1, dans laquelle la membrane antérieure est plus grande que la membrane annulaire postérieure.

12. Lentille selon la revendication 3, dans laquelle les parties étendues sont des haptiques et comportent une articulation adjacente à l'optique.

13. Lentille selon la revendication 1, dans laquelle la partie centrale solide postérieure a un rayon central fixe et le rayon antérieur central est déformable.

14. Lentille selon la revendication 1, dans laquelle une zone périphérique solide se prolonge pour se joindre au niveau d'une jonction articulée à la partie postérieure solide.
